# EUROPEAN PATENT APPLICATION

(11) **EP 4 778 631 A1**
(43) Date of publication of application: **22.07.2026**
(21) Application number: 24864051.8
(22) Date of filing: 16.04.2024
(51) Int. Cl.: B05B 7/02

(54) **ELECTRONIC ATOMIZATION DEVICE**

(30) Priority: 15.09.2023 CN 202322527834 U
(71) Applicant: Shenzhen Moore Vaporization Health & Medical Technology Co., Ltd., Shenzhen, Guangdong 518102 (CN)
(72) Inventor: ZHOU, Ruilong, Shenzhen, Guangdong 518102 (CN)
(74) Representative: Winter, Brandl - Partnerschaft mbB
(86) International application number: PCT/CN2024/088037
(87) International publication number: WO 2025/055325

(57) **Abstract**

This application provides an electronic atomization apparatus, including a liquid storage assembly and a nozzle module. The liquid storage assembly has a liquid storage cavity configured to store an atomizable substrate. The nozzle module is in communication with the liquid storage assembly. The nozzle module includes a nozzle and a mechanical valve. The mechanical valve is arranged in a fluid path between the liquid storage assembly and the nozzle. The mechanical valve can be configured to be in a first state based on a liquid pressure in the fluid path, to bring the liquid storage cavity into communication with the nozzle; or configured to be in a second state based on the liquid pressure in the fluid path, to block the fluid path between the liquid storage cavity and the nozzle. Through the design, the fluid path between the liquid storage cavity and the nozzle may be blocked through the mechanical valve at a moment atomization of the electronic atomization apparatus ends or within a preset time before the atomization ends, to avoid liquid spurting.

## Description

### CROSS-REFERENCE TO RELATED APPLICATIONS

This application claims priority to Chinese Patent Application No. 202322527834.5 filed on September 15, 2023, which is incorporated herein by reference in its entirety.

### TECHNICAL FIELD

This application relates to the field of atomization technologies, and in particular, to an electronic atomization apparatus.

### BACKGROUND

Currently, an electronic atomization apparatus on the market inhales an atomizable substrate out from a liquid storage bottle in a manner of rapidly flowing an air stream at a mouth of a nozzle to form a negative pressure, and then to atomize the atomizable substrate into small droplets and spray the small droplets out through the Venturi principle.

However, a problem of liquid spurting (splashing out of a liquid column) easily occurs at the nozzle of the electronic atomization apparatus at a moment atomization ends, thereby affecting use experience of a user.

### SUMMARY

This application provides an electronic atomization apparatus, which can resolve a problem that liquid spurting easily occurs at a moment atomization of the electronic atomization apparatus ends.

To resolve the foregoing problem, a technical solution provided in this application is to provide the electronic atomization apparatus, including: a liquid storage assembly, having a liquid storage cavity, where the liquid storage cavity is configured to store an atomizable substrate; and a nozzle module, in communication with the liquid storage assembly, and configured to atomize the atomizable substrate from the liquid storage assembly to form an aerosol, where the nozzle module includes a nozzle and a mechanical valve, and the mechanical valve is arranged in a fluid path between the liquid storage assembly and the nozzle module; and when a liquid pressure in the fluid path is greater than a preset threshold, the mechanical valve is configured to be in a first state, to bring the liquid storage cavity into communication with the nozzle, and when the liquid pressure in a first liquid guide pipeline is less than the preset threshold, the mechanical valve is configured to be in a second state, to block a fluid path between the liquid storage cavity and the nozzle.

In an embodiment, a volume of the liquid storage cavity is variable, and the liquid pressure in the fluid path is related to a volume compression rate of the liquid storage cavity.

In an embodiment, the mechanical valve includes a movable member, the movable member is opened under an action of the liquid pressure when the liquid pressure in the fluid path is greater than the preset threshold, to bring the liquid storage cavity into communication with the nozzle, and the movable member is closed under an action of an elastic force when the liquid pressure in the fluid path is less than the preset threshold, to block the fluid path between the liquid storage cavity and the nozzle.

In an embodiment, the fluid path includes a first liquid guide pipeline that brings the liquid storage cavity and the mechanical valve into communication, and a second liquid guide pipeline that brings the mechanical valve and the nozzle into communication; and when a liquid pressure in the first liquid guide pipeline is greater than the preset threshold, the mechanical valve is configured to be in the first state, and when the liquid pressure in the first liquid guide pipeline is less than the preset threshold, the mechanical valve is configured to be in the second state.

In an embodiment, the nozzle module includes the second liquid guide pipeline and a nozzle base, and the nozzle is mounted to the nozzle base. The mechanical valve includes: a first accommodating tank, mating with the nozzle base to form a first accommodating cavity, where the first accommodating cavity has a first liquid inlet and a first liquid outlet, the liquid storage cavity is brought into communication with the first accommodating cavity through the first liquid guide pipeline and the first liquid inlet, and the first accommodating cavity is brought into communication with the nozzle through the first liquid outlet and the second liquid guide pipeline; and the movable member, movably arranged in the first accommodating cavity, where when the liquid pressure in the first liquid guide pipeline is less than the preset threshold, the movable member blocks the first liquid inlet under the action of the elastic force, so as to block the fluid path between the liquid storage cavity and the nozzle; and when the liquid pressure in the first liquid guide pipeline is greater than the preset threshold, the movable member moves toward a direction away from the first liquid inlet under the action of the liquid pressure, to bring the liquid storage cavity into communication with the nozzle through the first accommodating cavity.

In an embodiment, the mechanical valve includes the movable member, and the movable member includes: a moving member, arranged in the first accommodating cavity; and an elastic member, arranged in the first accommodating cavity and connected between a cavity wall of the first accommodating cavity and the moving member, where when the liquid pressure in the first liquid guide pipeline is less than the preset threshold, the elastic member abuts the moving member against the first liquid inlet, to block the first liquid inlet; and when the liquid pressure in the first liquid guide pipeline is greater than the preset threshold, the elastic member is elastically deformed under the action of the liquid pressure, to allow the moving member to move toward the direction away from the first liquid inlet, so as to bring the liquid storage cavity into communication with the nozzle.

In an embodiment, the first accommodating tank has a first groove oriented towards the nozzle base, the nozzle base has a second groove oriented towards the first groove, and the first groove mates with the second groove to form the first accommodating cavity.

In an embodiment, an end of the first accommodating tank facing away from the first accommodating cavity has a third groove, the first liquid inlet is located on a bottom wall of the third groove, and at least part of the first liquid guide pipeline extends into the third groove and is brought into communication with the first accommodating cavity through the first liquid inlet.

In an embodiment, the mechanical valve further includes: the electronic atomization apparatus further includes: a first seal member, which is at least partially arranged between the third groove and the first liquid guide pipeline and is configured to hermetically connect the first accommodating tank to the first liquid guide pipeline.

In an embodiment, the liquid storage assembly includes: a second accommodating tank, having a second accommodating cavity and a second liquid outlet, where the second accommodating cavity is brought into communication with the mechanical valve through the second liquid outlet and the first liquid guide pipeline; a piston assembly, arranged in the second accommodating cavity, and slidable relative to the second accommodating cavity; and a driving mechanism, including a push rod, where the push rod is configured to drive the piston assembly to slide in the second accommodating cavity; and the piston assembly mates with the second accommodating cavity to form the liquid storage cavity, the volume of the liquid storage cavity is related to a position of the piston assembly in the second accommodating cavity, and the liquid pressure in the first liquid guide pipeline is related to a rate at which the push rod drives the piston assembly to slide in the second accommodating cavity.

In an embodiment, the electronic atomization apparatus further includes a control unit, connected to the driving mechanism, and configured to control the push rod in the driving mechanism to drive the piston assembly to slide in the second accommodating cavity, where when the electronic atomization apparatus is in an atomization state, the control unit drives the piston assembly to slide toward the second liquid outlet through the driving mechanism, so that the liquid pressure in the first liquid guide pipeline is continuously greater than the preset threshold, and then the mechanical valve is configured to be in the first state. When the electronic atomization apparatus is switched from the atomization state to a non-atomization state, the control unit controls the push rod to slide toward a direction away from the piston assembly, so that liquid pressure in the first liquid guide pipeline is less than the preset threshold, and then the mechanical valve is configured to be in the second state.

Different from the prior art, the electronic atomization apparatus provided in this application includes the liquid storage assembly and the nozzle module. The nozzle module includes the nozzle and the mechanical valve. The nozzle is configured to atomize the atomizable substrate in the liquid storage assembly. The mechanical valve is arranged in the fluid path between the liquid storage assembly and the nozzle. The mechanical valve can be configured to be in the first state based on a state in which the liquid pressure in the fluid path is greater than the preset threshold, to bring the liquid storage cavity into communication with the nozzle. Alternatively, the mechanical valve is configured to be in the second state based on a state in which the liquid pressure in the fluid path is greater than the preset threshold, to block the fluid path between the liquid storage cavity and the nozzle. Specifically, through this design, the fluid path between the liquid storage cavity and the nozzle may be blocked through the mechanical valve at a moment atomization of the electronic atomization apparatus ends or within a preset time before the atomization ends, so as to prevent the electronic atomization apparatus from spurting liquid at the moment atomization of the electronic atomization apparatus ends.

### BRIEF DESCRIPTION OF THE DRAWINGS

To describe technical solutions of embodiments of this application more clearly, drawings required for describing the embodiments are briefly described below. Apparently, the drawings in the following description show only some embodiments of this application, and a person of ordinary skill in the art can derive other drawings from the drawings without creative efforts.
FIG. 1 is a schematic structural diagram of an embodiment of an electronic atomization apparatus according to this application;
FIG. 2 is a schematic structural diagram of an embodiment of an electronic atomization apparatus with part of a housing removed according to this application;
FIG. 3 is a cross-sectional view of a liquid storage assembly and a nozzle module when a mechanical valve in the nozzle module is in a second state according to this application;
FIG. 4 is a cross-sectional view of a liquid storage assembly and a nozzle module when a mechanical valve in the nozzle module is in a first state according to this application;
FIG. 5 is an enlarged structural diagram of a region X in FIG. 3;
FIG. 6 is an enlarged structural diagram of a region Y in FIG. 4;
FIG. 7 is an exploded structural diagram of an embodiment of a nozzle module according to this application; and
FIG. 8 is a schematic structural diagram of an embodiment of a second accommodating tank according to this application.

### DETAILED DESCRIPTION

Technical solutions in embodiments of this application are clearly and completely described below with reference to accompanying drawings in the embodiments of this application. Apparently, the described embodiments are merely some rather than all of the embodiments of this application. All other embodiments obtained by a person of ordinary skill in the art based on the embodiments of this application without creative efforts fall within the protection scope of this application.

Terms "first", "second", and "third" in this application are merely intended for a purpose of description, and shall not be understood as an indication or implication of relative importance or implicit indication of a quantity of indicated technical features. Therefore, features defining "first", "second", and "third" may explicitly or implicitly include at least one of the features. In description of this application, "a plurality of" means at least two, such as two and three, unless otherwise specifically defined. All directional indications (for example, up, down, left, right, front, back) in the embodiments of this application are only used for explaining relative position relationships, movement situations, or the like between the various components in a specific posture (as shown in the accompanying drawings). If the specific posture changes, the directional indications change accordingly. In addition, terms "include", "have", and any variant thereof are intended to cover a non-exclusive inclusion. For example, a process, a method, a system, a product, or a device that includes a series of steps or units is not limited to the listed steps or units, and instead, further optionally includes a step or unit that is not listed, or further optionally includes another step or unit that is intrinsic to the process, the method, the product, or the device.

The "embodiment" mentioned in the specification means that particular features, structures, or characteristics described with reference to the embodiments may be included in at least one embodiment of this application. The phrase appearing at various locations in this specification does not necessarily indicate a same embodiment, and is not an independent or alternative embodiment exclusive to another embodiment. A person skilled in the art explicitly or implicitly understands that the embodiments described in the specification may be combined with other embodiments.

This application is described in detail below with reference to the accompanying drawings and embodiments.

The applicant finds that a problem of liquid spurting occurring at a moment atomization of the electronic atomization apparatus ends is as follows. Because the liquid pressure in the nozzle, the liquid guide pipeline, and the liquid storage cavity is not instantly released at the nozzle when the atomization ends, the internal pressure gradually decreases as the internal liquid gradually flows out, and the external flow of the liquid is stopped as the pressure gradually balances with an external environment. In this process, the liquid in the liquid storage cavity synchronously flows out and the liquid does not flow out unevenly, causing the problem of liquid spurting.

In addition, the liquid spurting may also cause the atomizable substrate to remain inside the nozzle, which may also cause the following problems. First, the atomizable substrate remaining inside the nozzle is in contact with air for a long time, easily breeding a microbe and causing the atomizable substrate to deteriorate due to being polluted by the microbe. Second, after water in the atomizable substrate remaining in the nozzle is evaporated, the atomizable substrate solidifies, and the solidified atomizable substrate easily blocks the nozzle, resulting in that the electronic atomization apparatus cannot normally atomize an aerosol when being used next time. Third, because another component such as an air pump have already stopped operating, the liquid in the abnormal process is not effectively atomized, causing waste and degrading user experience. In addition, even if part of the spurting liquid may be atomized in a manner of delaying a shut-down time of the air pump, another component, and the like, the part of the spurting liquid cannot be effectively atomized due to an extremely low subsequent liquid pressure, which may also reduce user experience.

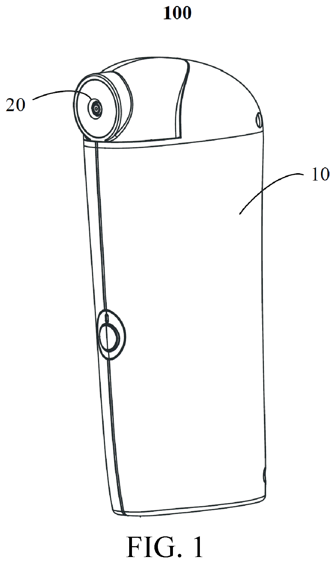

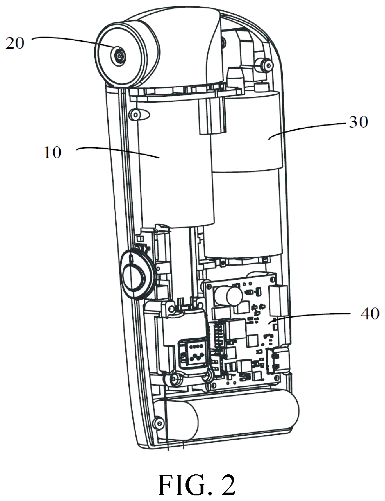

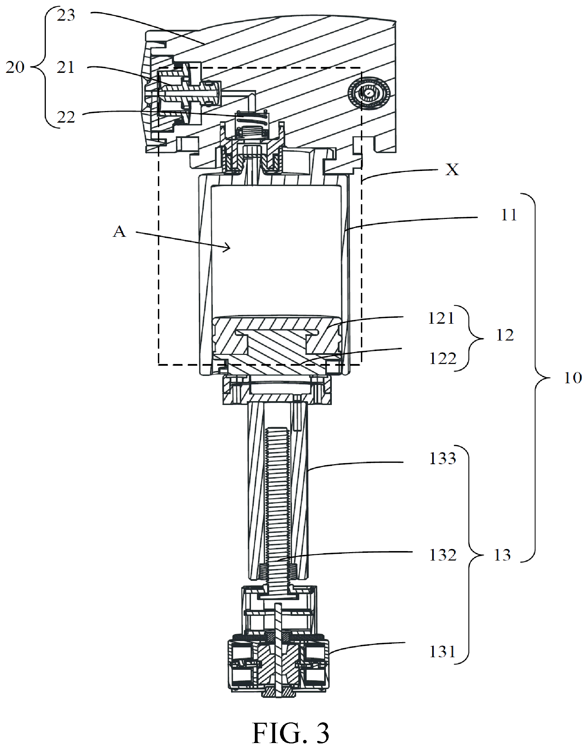

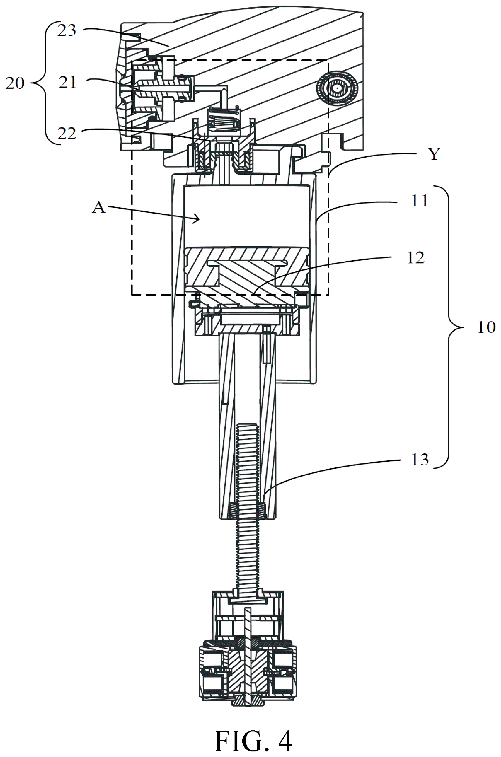

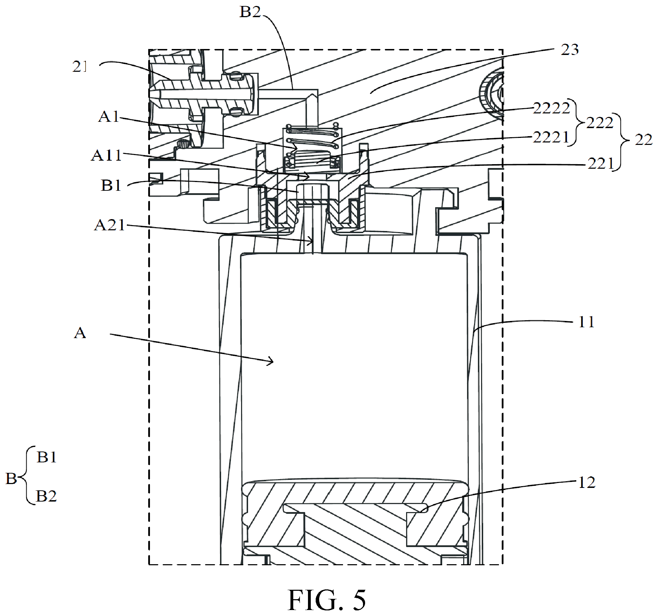

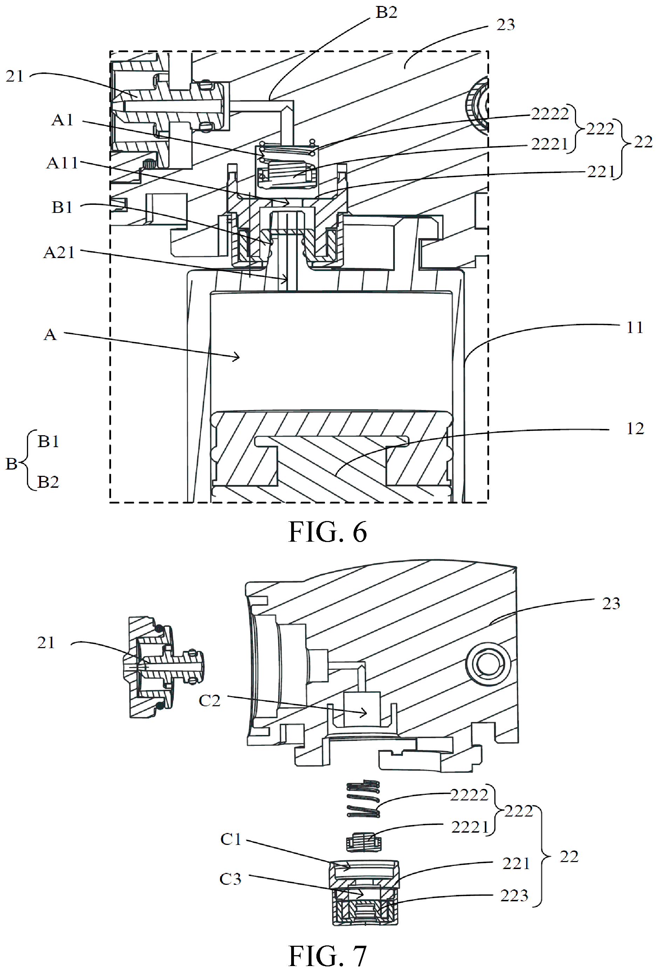

Referring to FIG. 1 to FIG. 7, FIG. 1 is a schematic structural diagram of an embodiment of an electronic atomization apparatus according to this application, FIG. 2 is a schematic structural diagram of an embodiment of an electronic atomization apparatus with part of a housing removed according to this application, FIG. 3 is a cross-sectional view of a liquid storage assembly and a nozzle module when a mechanical valve in the nozzle module is in a second state according to this application, FIG. 4 is a cross-sectional view of a liquid storage assembly and a nozzle module when a mechanical valve in the nozzle module is in a first state according to this application, FIG. 5 is an enlarged structural diagram of a region X in FIG. 3, FIG. 6 is an enlarged structural diagram of a region Y in FIG. 4, and FIG. 7 is an exploded structural diagram of an embodiment of a nozzle module according to this application.

To resolve the foregoing problem, embodiments of this application provide an electronic atomization apparatus 100, including a housing 101, and a liquid storage assembly 10 and a nozzle module 20 that are arranged in the housing 101.

The liquid storage assembly 10 has a liquid storage cavity A, and the liquid storage cavity A is configured to store an atomizable substrate. The atomizable substrate includes, but is not limited to, a medicinal liquid, a nutrient solution, a cosmetic liquid, an essential liquid, an essential lotion, a sterilizing liquid, an insecticide, a pesticide, and the like, so that the electronic atomization apparatus 100 to which the atomizable substrate is applied is applied to different fields such as a medical field, a cosmetic field, and an agricultural field.

The nozzle module 20 is in communication with the liquid storage assembly 10, and specifically, in communication with the liquid storage cavity A in the liquid storage assembly 10, which is configured to atomize an atomizable substrate from the liquid storage assembly 10 to form an aerosol.

Referring to FIG. 2, in an embodiment, the electronic atomization apparatus 100 may further include an air supply unit 30. The air supply unit 30 is configured to provide a high-pressure air for the nozzle module 20. The high-pressure air may shear the atomizable substrate that enters the nozzle module 20 into atomization particles having a suitable particle size, that is, the aerosol, and carry the aerosol out of a spraying port of the nozzle module 20. The air supply unit 30 may be an air pump.

In an embodiment, the nozzle module 20 may further include an atomization element (not shown in the figure). The atomization element is configured to atomize the atomizable substrate to form the aerosol. The atomization element may be an ultrasonic atomization element, an electric heating atomization element, a two-phase flow atomization nozzle, or the like in the prior art. Specifically, as shown in FIG. 3, in this embodiment, the two-phase flow atomization nozzle is adopted. In other words, an aerosol is generated through the interaction between a high-speed airflow and liquid.

Referring to FIG. 3 to FIG. 7, in an embodiment, the nozzle module 20 includes a nozzle 21 and a mechanical valve 22. The nozzle 21 is configured to atomize the atomizable substrate, and the mechanical valve 22 is arranged in a fluid path B between the liquid storage assembly 10 and the nozzle 21. Specifically, the mechanical valve 22 is configured to turn on or shut off the fluid path B between the liquid storage assembly 10 and the nozzle module 20. For example, when the electronic atomization apparatus 100 is in an atomization state, the mechanical valve 22 is configured to turn on the fluid path B between the liquid storage assembly 10 and the nozzle, so as to allow the liquid storage cavity A to supply liquid to the nozzle 21 to atomize and generate the aerosol. Alternatively, when the electronic atomization apparatus 100 is in a non-atomization state, the mechanical valve 22 is configured to shut off the fluid path B between the liquid storage assembly 10 and the nozzle 21, so as to prohibit the liquid storage cavity A from supplying liquid to the nozzle 21, thereby avoiding problems such as liquid leakage and liquid spurting.

In this embodiment of this application, the volume of the liquid storage cavity A is variable. It may be understood that a variable part of the volume of the liquid storage cavity A specifically refers to a part of a space that actually stores an atomizable substrate. The liquid storage cavity A having a variable volume facilitates transferring the liquid outward at a certain pressure, while ensuring good consistency of liquid supply. The liquid pressure in the fluid path B is related to a volume compression rate of the liquid storage cavity A, and the compression rate is a change amount of the volume per unit time. An implementation in which the volume of the liquid storage cavity A is variable may be a liquid storage cavity A of a piston type, and the volume of the liquid storage cavity A is compressed through a slidable piston. In addition, a stroke speed of the piston or the volume compression rate of the liquid storage cavity A is adjusted, so as to change a liquid pressure in the fluid path B connected to the liquid storage cavity A. Alternatively, an implementation in which the volume of the liquid storage cavity A is variable may be an elastic liquid storage cavity A. A cavity wall of the elastic liquid storage cavity A is elastic and can be contracted or expanded, so as to change the volume of the liquid storage cavity A, thereby changing the liquid pressure in the fluid path B connected to the liquid storage cavity A. It may be understood that the compression rate is 0, that is, the liquid storage cavity does not provide liquid to the outside. In this case, the volume of the liquid storage cavity A should not change. Meanwhile, no positive pressure that pushes the liquid to flow exists in the fluid path B.

In this embodiment of this application, the mechanical valve 22 is switched to different states based on the liquid pressure in the fluid path B. For example, when the liquid pressure in the fluid path B is greater than a preset threshold, the mechanical valve 22 is configured to be in a first state, to bring the liquid storage cavity A into communication with the nozzle 21; and when the liquid pressure in the fluid path B is less than the preset threshold, the mechanical valve 22 is configured to be in a second state, to block a fluid path between the liquid storage cavity A and the nozzle 21. Specifically, through the design, the liquid pressure in the fluid path B that is in communication with the liquid storage cavity A may be changed by changing the volume compression rate of the liquid storage cavity A or the stroke speed of the piston assembly at a moment when atomization of the electronic atomization apparatus 100 ends or within a preset time before the atomization ends, so that the mechanical valve 22 is switched to be in the second state, and the fluid path B between the liquid storage cavity A and the nozzle 21 is blocked, to prevent the electronic atomization apparatus 100 from spurting liquid at the moment when the atomization ends, thereby preventing the spurting liquid from generating another associated problem (for example, deterioration of a residual atomizable substrate in the nozzle 21 or blocking of the nozzle 21).

In an embodiment, the mechanical valve 22 includes a movable member 222. The movable member 222 is opened under an action of the liquid pressure when the liquid pressure in the fluid path B is greater than the preset threshold, to bring the liquid storage cavity A into communication with the nozzle 21, and the movable member 222 is closed under an action of an elastic force when the liquid pressure in the fluid path B is less than the preset threshold, to block the fluid path B between the liquid storage cavity A and the nozzle 21.

Specifically, the preset threshold is related to the elasticity of the movable member 222. A direction of the elastic force of the movable member 222 is opposite to a direction of an acting force (that is, a hydraulic pressure) that is applied to the movable member 222 by the liquid pressure in the fluid path B. The movable member 222 is configured to open or block the fluid path B under the action of the elastic force thereof and the hydraulic pressure in the fluid path B. In other words, the mechanical valve 22 is opened in a case that the hydraulic pressure generated when the liquid pressure is applied to the movable member 222 is greater than the elastic force of the movable member 222. The mechanical valve 22 is closed in a case that the hydraulic pressure generated when the liquid pressure is applied to the movable member 222 is less than the elastic force of the movable member 222.

In an embodiment, the fluid path B includes a first liquid guide pipeline B1 that brings the liquid storage cavity A and the mechanical valve 22 into communication, and a second liquid guide pipeline B2 that brings the mechanical valve 22 and the nozzle 21 into communication. It may be understood that the mechanical valve 22 is brought into communication with the liquid storage cavity A through the first liquid guide pipeline B1. Therefore, a liquid pressure in the liquid storage cavity A is in positive correlation with a liquid pressure in the first liquid guide pipeline B1, and turning-on/shutting-off of the mechanical valve 22 is associated with the liquid pressure in the first liquid guide pipeline B1. As a result, when the liquid pressure in the first liquid guide pipeline B1 is greater than the preset threshold, that is, the hydraulic pressure generated by the liquid in the first liquid guide pipeline B1 acting on the movable member 22 is greater than the elastic force of the movable member 22, the movable member 222 is opened under the action of the hydraulic pressure, and the mechanical valve 22 is configured to be in the first state, so that the liquid storage cavity A is brought into communication with the nozzle 21. When the hydraulic pressure generated by the liquid in the first liquid guide pipeline B1 acting on the movable member 22 is less than the preset threshold, that is, the hydraulic pressure in the first liquid guide pipeline B1 is less than the elastic force of the movable member 22, the movable member is closed under the action of the elastic force, and the mechanical valve 22 is configured to be in the second state, so as to block the fluid path B between the liquid storage cavity A and the nozzle 21. It may be understood that the liquid converts the liquid pressure into the hydraulic pressure acting on the movable member 22 through an equivalent contact area between the liquid and the movable member 22. Generally, the equivalent contact surface does not greatly change as the equivalent contact surface is turned on or shut off. In other words, the equivalent contact surface has a constant value. Therefore, the magnitude of the hydraulic pressure is in linearly positive correlation with the magnitude of the liquid pressure. In this application, the hydraulic pressure acting on the movable member 22 may be equivalent to the liquid pressure through conversion. Therefore, the hydraulic pressure is not distinguished from the liquid pressure.

In some embodiments, the nozzle module 20 is detachably connected to the liquid storage assembly 10, and/or the mechanical valve 22 is detachably connected to the nozzle 21.

Specifically, the foregoing components may be easily replaced or repaired separately through a detachable connection between the nozzle module 20 and the liquid storage assembly 10 and a detachable connection between the mechanical valve 22 and the nozzle 21, thereby reducing use costs of the user.

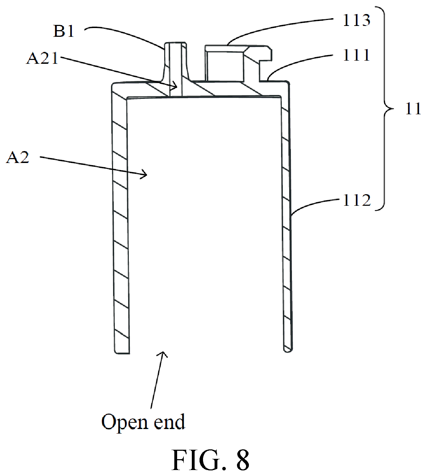

With reference to FIG. 3 and FIG. 8, FIG. 8 is a schematic structural diagram of an embodiment of a second accommodating tank according to this application. In an embodiment, the liquid storage assembly 10 includes a second accommodating tank 11, a piston assembly 12, and a driving mechanism 13.

The second accommodating tank 11 has a second accommodating cavity A2 and a second liquid outlet A21. The second accommodating cavity A2 is brought into communication with the mechanical valve 22 through the second liquid outlet A21 and the first liquid guide pipeline B1. The piston assembly 12 is arranged in the second accommodating cavity A2 and is slidable relative to the second accommodating cavity A2. The piston assembly 12 mates with the second accommodating cavity A2 to form the liquid storage cavity A, and a volume of the liquid storage cavity A is related to a position of the piston assembly 12 in the second accommodating cavity A2. The driving mechanism 13 includes a push rod 133. The push rod 133 is configured to drive the piston assembly 12 to slide in the second accommodating cavity A2, so as to change the volume of the liquid storage cavity A. The liquid pressure in the first liquid guide pipeline B1 is related to a rate at which the push rod 133 drives the piston assembly 12 to slide in the second accommodating cavity A2 (equivalent to a rate at which the volume of the liquid storage cavity A is compressed).

With reference to FIG. 2, in an embodiment, the electronic atomization apparatus 100 further includes a control unit 40. The control unit 40 is connected to the driving mechanism 13, and is configured to control the driving mechanism 13 to drive the piston assembly 13 to slide in the second accommodating cavity A2, so as to change the volume of the liquid storage cavity A.

For example, when the electronic atomization apparatus 100 is in an atomization state, the control unit 40 drives the piston assembly 13 to slide toward the second liquid outlet A21 through the push rod 133, so that the liquid pressure in the first liquid guide pipeline B1 is continuously greater than the preset threshold, and the mechanical valve 22 is configured to be in the first state, to ensure continuous liquid supply from the liquid storage cavity A to the nozzle 21.

When the electronic atomization apparatus 100 is switched from the atomization state to a non-atomization state, the control unit 40 controls the push rod 133 to slide toward a direction away from the piston assembly 12, and the volume of the liquid storage cavity A is not compressed by the piston assembly 12, so that the liquid pressure in the first liquid guide pipeline B1 is less than the preset threshold, and the mechanical valve 22 is configured to be in the second state, and the fluid path B between the liquid storage cavity A and the nozzle 21 is shut off, thereby avoiding a problem of liquid spurting caused by a back pressure on the nozzle 21 at a moment when the electronic atomization apparatus 100 is powered off.

In an embodiment, in response to receiving an atomization signal by the control unit 40, for example, the control unit 40 receives an inhalation signal of the user, the control unit 40 receives a power-on signal of the electronic atomization apparatus 100, or the control unit 40 receives an atomization mode signal of the electronic atomization apparatus 100, the control unit 40 controls the push rod 133 to drive the piston assembly 12 to move in the second accommodating cavity A2 toward a direction close to the second liquid outlet A21, to compress the volume of the liquid storage cavity A, so that the liquid pressure in the first liquid guide pipeline B1 is continuously greater than the preset threshold, and the mechanical valve 22 is configured to be in the first state, thereby ensuring continuous liquid supply of the liquid storage cavity A to the nozzle 21.

In response to stopping the atomization signal by the control unit 40, for example, the control unit 40 receives a signal that the user stops the inhalation, the control unit 40 receives a power-off signal of the electronic atomization apparatus 100, or he control unit 40 receives a signal of switching the electronic atomization apparatus 100 to a non-atomization mode, the control unit 40 controls the push rod 133 to slide toward a direction away from the piston assembly 12, so that the volume of the liquid storage cavity A is not compressed, the liquid pressure in the first liquid guide pipeline B1 is less than the preset threshold, and the mechanical valve 22 is quickly switched from the first state to the second state, thereby avoiding the problem of liquid spurting generated by the electronic atomization apparatus 100.

Still referring to FIG. 3 and FIG. 8, in an embodiment, the second accommodating tank 11 includes a top wall 111 and an annular side wall 112. An end of the annular side wall 112 facing away from the top wall 111 is an open end. The top wall 111 and the annular side wall 112 define the second accommodating cavity A2. The piston assembly 12 covers the open end to mate with the second accommodating cavity A2 to form the liquid storage cavity A with a variable volume. The structure has the following beneficial effects. The piston assembly 12 is facilitated to be mounted into the second accommodating cavity A2 from the open end, and the second accommodating tank 11 has a simple structure and is easy to manufacture.

In this embodiment, the second liquid outlet A21 may be arranged in the top wall 111. Alternatively, the second liquid outlet A21 may be arranged on an end of the annular side wall 112 close to the top wall 111, so that when the piston assembly 12 moves toward the second liquid outlet A21, the atomizable substrate in the liquid storage cavity A can be pushed out of the second liquid outlet A21 to the greatest extent, thereby avoiding waste.

Further, a limiting member (not shown in the figure) may be arranged on the open end. The limiting member is configured to limit the piston assembly 12 when the piston assembly 12 moves to the open end, to prevent the piston assembly 12 from disengaging from the second accommodating cavity A2.

Still referring to FIG. 8, in an embodiment, a snap-fit portion 113 is further arranged on the second accommodating tank 11. The snap-fit portion 113 is configured to be snap-fitted with the nozzle module 20 or another component in the electronic atomization apparatus 100 when the liquid storage assembly 10 is mounted into the electronic atomization apparatus 100, so as to achieve an effect of fixing the liquid storage assembly 10.

Still referring to FIG. 3 and FIG. 8, in an embodiment, the piston assembly 12 includes a second seal member 121 and a mounting base 122. The second seal member 121 mates with the second accommodating cavity A2 to form the liquid storage cavity A. The mounting base 122 is arranged on an end of the seal member facing away from the liquid storage cavity A and is connected to the second seal member 121, and an end of the mounting base 122 facing away from the second seal member 121 is configured to be connected to the driving mechanism 13.

Specifically, the second seal member 121 is arranged on a side of the mounting base 122 close to the second liquid outlet A21, and a material of the second seal member 121 may be silicon or rubber, so as to achieve an effect of sealing the liquid storage cavity A, thereby avoiding leakage of the atomizable substrate in the liquid storage cavity A.

Still referring to FIG. 3, in an embodiment, the driving mechanism 13 includes a motor 131, a pull rod 132, and a push rod 133. The motor 131 is electrically connected to the control unit 40. The pull rod 132 is connected to a driving shaft of the motor 131. An external thread is arranged on one of the pull rod 132 and the push rod 133, and an internal thread is arranged on the other of the pull rod and the push rod. The pull rod 132 is connected into the push rod 133 through a threaded connection by using the external thread and the internal thread. Specifically, the control unit 40 is configured to control the motor 131 to operate. The motor 131 may drive the pull rod 132 to rotate during operating. In addition, because the pull rod 132 is connected into the push rod 133 through a threaded connection, the push rod 133 may move up and down in a direction perpendicular to the rotation direction when the pull rod 132 is rotated, so that the push rod may drive the piston assembly 12 to slide in the second accommodating cavity A2 toward the second liquid outlet A21, or drive the push rod 133 to move away from the piston assembly 12 to detach from the piston assembly 12.

Referring to FIG. 5 to FIG. 7, in an embodiment, the nozzle module 20 includes a nozzle base 23 and a second liquid guide pipeline B2. The nozzle 21 is mounted to the nozzle base 23, and is brought into communication with the mechanical valve 22 through the second liquid guide pipeline B2.

The mechanical valve 22 further includes the first accommodating tank 221.

The first accommodating tank 221 mates with the nozzle base 23 to form the first accommodating cavity A1. The first accommodating cavity A1 may be at least one of the first accommodating tank 221 and the nozzle base 23 with a groove facing each other, so as to mate to form the first accommodating cavity A1. The first accommodating cavity A1 has a first liquid inlet A11 and a first liquid outlet A12. The liquid storage cavity A is brought into communication with the first accommodating cavity A1 through the second liquid outlet A21, the first liquid guide pipeline B1, and the first liquid inlet A11. The first accommodating cavity A1 is brought into communication with the nozzle 21 through the first liquid outlet A12 and the second liquid guide pipeline B2. The movable member 222 is movably arranged in the first accommodating cavity A1, and is configured to expose the first liquid inlet A11 when the mechanical valve 22 is in the first state, so that the liquid storage cavity A is in communication with the first accommodating cavity A1, and is configured to block the first liquid inlet A11 when the mechanical valve 22 is in the second state, so as to block the fluid path B between the liquid storage cavity A and the first accommodating cavity A1.

Specifically, when the liquid pressure in the first liquid guide pipeline B1 is less than the preset threshold, that is, the elastic force of the movable member 222 is greater than the liquid pressure in the first liquid guide pipeline B1, the movable member 222 blocks the first liquid inlet A11 under the action of the elastic force, so as to block the fluid path B between the liquid storage cavity A and the nozzle 21. When the liquid pressure in the first liquid guide pipeline B1 is greater than the preset threshold, that is, the elastic force of the movable member 222 is less than the hydraulic pressure in the first liquid guide pipeline B1, the movable member 222 moves toward a direction away from the first liquid inlet A11 under the action of the hydraulic pressure in the first liquid guide pipeline B1, so that the liquid storage cavity A is brought into communication with the nozzle 21 through the first accommodating cavity A1.

In a specific embodiment, the movable member 222 includes a moving member 2221 and an elastic member 2222.

The moving member 2221 is arranged in the first accommodating cavity A1. The elastic member 2222 is arranged in the first accommodating cavity A1 and is connected between a cavity wall of the first accommodating cavity A1 and the moving member 2221. Specifically, in this embodiment, the preset threshold may be elastic potential energy of the elastic member 2222 when the elastic member 2222 abuts the moving member 2221 against the first liquid inlet A11. The elastic member 2222 can be further elastically deformed based on the liquid pressure in the first liquid guide pipeline B1, so that the moving member 2221 connected to the elastic member blocks or exposes the first liquid inlet A11, thereby turning on or shutting off the fluid path B between the liquid storage cavity A and the nozzle 21.

Specifically, when the liquid pressure in the first liquid guide pipeline B1 is less than the preset threshold, the elastic member 2222 abuts the moving member 2221 against the first liquid inlet A11, to block the first liquid inlet A11, thereby blocking the fluid path B between the liquid storage cavity A and the nozzle 21. When the liquid pressure in the first liquid guide pipeline B1 is greater than the preset threshold, the elastic member 2222 is elastically deformed under the action of the hydraulic pressure, to allow the moving member 2221 to move toward a direction away from the first liquid inlet A11, so as to bring the liquid storage cavity A into communication with the nozzle 21.

With reference to FIG. 3 and FIG. 7, in an embodiment, the first accommodating tank 221 has a first groove C1 facing the nozzle base 23, the nozzle base 23 has a second groove C2 facing the first groove C1, and the first groove C1 mates with the second groove C2 to form the first accommodating cavity A1. Specifically, the first accommodating tank 221 has the first groove C1, the nozzle base 23 has the second groove C2, and the first groove C1 mates with the second groove C2 to form the first accommodating cavity A1. Compared with dedicatedly arranging the first accommodating cavity A1 in the first accommodating tank 221 or the nozzle base 23, a manufacturing process of the first accommodating cavity A1 may be simplified.

With reference to FIG. 3 and FIG. 7, in an embodiment, an end of the first accommodating tank 221 facing away from the first accommodating cavity A1 has a third groove C3, the first liquid inlet A11 is located on a bottom wall of the third groove C3, at least part of the first liquid guide pipeline B1 extends into the third groove C3, to improve reliability of the connection between the first liquid guide pipeline B1 and the first accommodating tank 221, and the first liquid guide pipeline B1 is brought into communication with the first accommodating cavity A1 through the first liquid inlet A11.

In an embodiment, the mechanical valve 22 further includes a first seal member 223. The first seal member 223 is at least partially arranged between the third groove C3 and the first liquid guide pipeline B1, and is configured to hermetically connect the first liquid guide pipeline B1 to the first accommodating tank 221. A material of the first seal member 223 may be silicon or rubber. The first seal member 223 can avoid leakage of an atomizable substrate at a joint between the first liquid guide pipeline B1 and the first accommodating tank 221, thereby avoiding affecting operation of the electronic atomization apparatus 100 and avoiding problems such as sanitation.

Specifically, the electronic atomization apparatus 100 provided in this application includes the liquid storage assembly 10 and the nozzle module 20. The nozzle module 20 is configured to atomize the atomizable substrate provided by the liquid storage assembly 10. The nozzle module 20 includes the nozzle 21 and the mechanical valve 22. The mechanical valve 22 is arranged on the fluid path B between the liquid storage assembly 10 and the nozzle 21. The mechanical valve 22 is switched to different states based on magnitudes of the liquid pressure in the fluid path B and the preset threshold. Specifically, through this design, the liquid pressure in the fluid path B may be changed at a moment when atomization of the electronic atomization apparatus 100 ends or within the preset time before the atomization ends, so that the mechanical valve 22 is switched to the second state, and the fluid path B between the liquid storage cavity A and the nozzle 21 is blocked, so as to avoid the electronic atomization apparatus 100 from spurting liquid at the moment when the atomization ends, thereby preventing the liquid spurting from generating another associated problem.

The foregoing descriptions are merely implementations of this application, and the patent scope of this application is not limited thereto. All equivalent structure or equivalent process changes made according to the content of this specification and accompanying drawings in this application or by directly or indirectly applying this application in other related technical fields shall fall within the protection scope of this application.

## Claims

1. An electronic atomization apparatus, comprising:
a liquid storage assembly, having a liquid storage cavity, wherein the liquid storage cavity is configured to store an atomizable substrate; and
a nozzle module, in communication with the liquid storage assembly, and configured to atomize the atomizable substrate from the liquid storage assembly to form an aerosol, wherein the nozzle module comprises a nozzle and a mechanical valve, and the mechanical valve is arranged in a fluid path between the liquid storage assembly and the nozzle; and
when a liquid pressure in the fluid path is greater than a preset threshold, the mechanical valve is configured to be in a first state, to bring the liquid storage cavity into communication with the nozzle, and when the liquid pressure in the fluid path is less than the preset threshold, the mechanical valve is configured to be in a second state, to block a fluid path between the liquid storage cavity and the nozzle.

2. The electronic atomization apparatus of claim 1, wherein
a volume of the liquid storage cavity is variable, and the liquid pressure in the fluid path is related to a volume compression rate of the liquid storage cavity.

3. The electronic atomization apparatus of claim 1, wherein the mechanical valve comprises a movable member, the movable member is opened under an action of the liquid pressure when the liquid pressure in the fluid path is greater than the preset threshold, to bring the liquid storage cavity into communication with the nozzle, and the movable member is closed under an action of an elastic force when the liquid pressure in the fluid path is less than the preset threshold, to block the fluid path between the liquid storage cavity and the nozzle.

4. The electronic atomization apparatus of any of claims 1 to 3, wherein the fluid path comprises a first liquid guide pipeline that brings the liquid storage cavity and the mechanical valve into communication, and a second liquid guide pipeline that brings the mechanical valve and the nozzle into communication; and
when a liquid pressure in the first liquid guide pipeline is greater than the preset threshold, the mechanical valve is configured to be in the first state, and when the liquid pressure in the first liquid guide pipeline is less than the preset threshold, the mechanical valve is configured to be in the second state.

5. The electronic atomization apparatus of claim 4, wherein the nozzle module comprises the second liquid guide pipeline and a nozzle base, and the nozzle is mounted to the nozzle base; and
the mechanical valve comprises:
a first accommodating tank, mating with the nozzle base to form a first accommodating cavity, wherein the first accommodating cavity has a first liquid inlet and a first liquid outlet, the liquid storage cavity is brought into communication with the first accommodating cavity through the first liquid guide pipeline and the first liquid inlet, and the first accommodating cavity is brought into communication with the nozzle through the first liquid outlet and the second liquid guide pipeline; and
the movable member, movably arranged in the first accommodating cavity, wherein
when the liquid pressure in the first liquid guide pipeline is less than the preset threshold, the movable member blocks the first liquid inlet under the action of the elastic force, so as to block the fluid path between the liquid storage cavity and the nozzle; and
when the liquid pressure in the first liquid guide pipeline is greater than the preset threshold, the movable member moves toward a direction away from the first liquid inlet under the action of the liquid pressure, to bring the liquid storage cavity into communication with the nozzle through the first accommodating cavity.

6. The electronic atomization apparatus of claim 5, wherein the mechanical valve comprises the movable member, and the movable member comprises:
a moving member, arranged in the first accommodating cavity; and
an elastic member, arranged in the first accommodating cavity and connected between a cavity wall of the first accommodating cavity and the moving member, wherein
when the liquid pressure in the first liquid guide pipeline is less than the preset threshold, the elastic member abuts the moving member against the first liquid inlet, to block the first liquid inlet; and
when the liquid pressure in the first liquid guide pipeline is greater than the preset threshold, the elastic member is elastically deformed under the action of the liquid pressure, to allow the moving member to move toward the direction away from the first liquid inlet, so as to bring the liquid storage cavity into communication with the nozzle.

7. The electronic atomization apparatus of claim 5, wherein the first accommodating tank has a first groove oriented towards the nozzle base, the nozzle base has a second groove oriented towards the first groove, and the first groove mates with the second groove to form the first accommodating cavity.

8. The electronic atomization apparatus of claim 7, wherein an end of the first accommodating tank facing away from the first accommodating cavity has a third groove, the first liquid inlet is located on a bottom wall of the third groove, and at least part of the first liquid guide pipeline extends into the third groove and is brought into communication with the first accommodating cavity through the first liquid inlet.

9. The electronic atomization apparatus of claim 8, wherein the mechanical valve further comprises:
a first seal member, at least partially arranged between the third groove and the first liquid guide pipeline, and configured to hermetically connect the first accommodating tank to the first liquid guide pipeline.

10. The electronic atomization apparatus of claim 4, wherein the liquid storage assembly comprises:
a second accommodating tank, having a second accommodating cavity and a second liquid outlet, wherein the second accommodating cavity is brought into communication with the mechanical valve through the second liquid outlet and the first liquid guide pipeline;
a piston assembly, arranged in the second accommodating cavity, and slidable relative to the second accommodating cavity; and
a driving mechanism, comprising a push rod, wherein the push rod is configured to drive the piston assembly to slide in the second accommodating cavity; and
the piston assembly mates with the second accommodating cavity to form the liquid storage cavity, the volume of the liquid storage cavity is related to a position of the piston assembly in the second accommodating cavity, and the liquid pressure in the first liquid guide pipeline is related to a rate at which the push rod drives the piston assembly to slide in the second accommodating cavity.
